# EUROPEAN PATENT APPLICATION

(11) **EP 4 321 277 A1**
(43) Date of publication of application: **14.02.2024**
(21) Application number: 21936042.7
(22) Date of filing: 08.04.2021
(51) Int. Cl.: B22F 1/00, B22F 9/00

(54) **SILICON-COMPOUND-COATED METAL MAGNESIUM PARTICLES**

(71) Applicant: M. Technique Co., Ltd., Izumi-shi, Osaka 594-1144 (JP)
(72) Inventor: ENOMURA Masakazu, Izumi-shi, Osaka 594-1144 (JP); OKAWA Hideki, Izumi-shi, Osaka 594-1144 (JP)
(74) Representative: Marks & Clerk LLP
(86) International application number: PCT/JP2021/014956
(87) International publication number: WO 2022/215238

(57) **Abstract**

The present invention relates to silicon compound coated metal magnesium particles in which at least a part of a surface of the metal magnesium particles is coated with the silicon compound, wherein an average particle diameter of the silicon compound coated metal magnesium particles is 5 nm or more and 500 nm or less, and a thickness of the silicon compound coating is 1 nm or more and 50 nm or less. The silicon compound coated metal magnesium particles of the present invention are easy to handle in the atmosphere, and have excellent oxidation resistance, and further the properties expected of the nano-size metal magnesium particles are improved at a maximum, or the properties are controlled by supplementing the properties.

## Description

### TECHNICAL FIELD

The present invention relates to silicon compound coated metal magnesium particles.

### BACKGROUND ART

Magnesium has various properties such as the lightest weight among metals, high specific strength, excellent vibration absorption, recyclability, heat dissipation, electromagnetic shielding capacity, machinability, etc., and abundant reserves. Taking advantage of such properties, applications thereof such as a secondary battery material and a biomedical implant material are currently underway. Regarding a secondary battery material, since charging and discharging are frequently repeated in lithium-ion batteries, research and development of secondary batteries with improved performance in the number of repeated charging and discharging cycles are underway. There is a demand for secondary batteries based on an element that is more abundant on earth, from the view point of effective resource utilization. Among them, metal magnesium is known to be promising as a secondary battery material that can also solve the problem of resource depletion (Patent Literature 1). In addition, magnesium has a low density alongside aluminum among practical metals, and magnesium has no adverse effects on the living body and has a relatively corrosive feature. In view of utilizing these features, study of magnesium for a biomedical implant material is also under consideration. Since magnesium is essential for living organisms, and corrodes and dissolves in the living body, magnesium is attracting attention as a metal that reduces stress on a patient without having to remove the implant material again. Furthermore, magnesium and a magnesium alloy are also known as a hydrogen storage metal and a hydrogen storage alloy.

Regarding a negative electrode material of magnesium ion secondary batteries, Patent Literature 1 discloses metal magnesium particles in which an allotrope of carbon is in contact with the surface of the metal magnesium. Patent Literature 1 describes carbon having a graphene structure other than diamond as an allotrope of carbon. Since a carbon allotrope having a graphene structure is an electrical conductor, magnesium ions in the electrolyte of a magnesium ion secondary battery must originally be reduced on the surface of metal magnesium, but the particles of Patent Literature 1 are reduced to metal magnesium even on the graphene surface, after receiving electrons. However, since magnesium ions are reduced via carbon on the surface of metal magnesium, there is a problem in that the efficiency of reducing magnesium ions to metal magnesium decreases, resulting in decrease of current efficiency in a negative electrode material of magnesium ion secondary batteries.

Patent Literature 2 discloses metal magnesium powders whose surface is coated with silicon oxide powders. However, the metal magnesium powders of Patent Literature 2 are exclusively for use in flux-cored wires for arc welding in which flux is filled in a metal outer sheath. The particle size of the metal magnesium powders are several tens of µm, and the specific surface area thereof is extremely small compared with nano-size particles used for a secondary battery material. Since the charging and discharging reactions of a secondary battery proceed on the surface of the metal magnesium particles, even if these particles are applied to the negative electrode material of magnesium ion secondary batteries, the utilization efficiency as the negative electrode of the secondary battery will be low, and battery performance improvement thereby cannot be expected.

Furthermore, it is known that metal magnesium can be applied to an in vivo implant material in addition to the above mentioned negative electrodes of magnesium ion secondary batteries, as a product utilizing the property that metal magnesium can be dissolved as a magnesium ion. Patent Literature 3 discloses forming a composite coating containing a biodegradable polymer and a hydroxide on a substrate made of magnesium or a magnesium alloy. Patent Literature 3 discloses that by using this material, an implant material used as an embedded metal fitting in surgeries such as fracture treatment dissolve in the living body, eliminating the need to remove the implant material again after healing, thereby reducing the burden on the patient.

Patent Literature 3 describes that the composite coating consisting of a biodegradable polymer and magnesium hydroxide is applied as an anticorrosive coating on the surface of the magnesium substrate, in order to prevent magnesium from eluting into the living body and failing to function as an implant material before the affected area heals. Patent Literature 3 describes that the composite coating consisting of a biodegradable polymer and magnesium hydroxide can be formed by subjecting a magnesium substrate to hydrothermal treatment in an aqueous solution of a biodegradable polymer. Since these coatings are applied as a posttreatment to the surface-treated magnesium substrate, binding strength between the magnesium hydroxide generated on the surface of the magnesium substrate and the biodegradable polymer is expected to be varied. That is, Patent Literature 3 discloses L-aspartic acid as a biodegradable polymer, and that it is thought that a hydrogen bond is formed between the oxygen atom of the carbonyl group of L-aspartic acid and the hydrogen atom of the hydroxyl group of magnesium hydroxide to obtain the bonding strength. However, since the bonding strength of hydrogen bond is likely to be affected by the pH of a body fluid in the living body, the bonding strength between L-aspartic acid and magnesium hydroxide tends to vary. As a result, a problem is assumed that the dissolution rate of magnesium into the living body becomes non-uniform, and the implant material tends to remain non-uniformly. Next, regarding a device that utilizes a material such as a photocatalyst and surface plasmons of metal particles, etc., conventionally, precious metals such as silver and gold have been used as the metal, but its popularization have been hindered because of restrictions on characteristics and high cost caused by using a precious metal.

On the other hand, Patent Literatures 4 and 5 by the applicant of the present application, disclose the silicon compound coated metal particles obtained by using the fluid processing apparatus described in Patent Literature 6, which is composed of processing surfaces being capable of approaching to and separating from each other and rotating relative to each other. However, with respect to nano-size metal magnesium, for example, metal magnesium reacts with water and dissolves, when using pure water as a reaction solvent, or metal magnesium reacts with an alcohol such as methanol to produce magnesium alkoxide, when using an alcohol. Metal magnesium cannot be precipitated as metal particles, when using such solvents. Thus, it was not easy to select a solvent, and it was very difficult to coat nano-size metal magnesium particles with a silicon compound. In addition, since metal magnesium is easily oxidized in the atmosphere, even if metal magnesium particles are obtained, they will be immediately oxidized unless the particles have a strictly controlled surface condition. Accordingly, nano-size metal magnesium particles that could be used for a secondary battery, an implant, etc. have not been actually known.

As a result, metal magnesium is regarded as promising as a negative electrode material of secondary batteries with high energy efficiency, as a material for an implant, as various materials such as a photocatalyst that takes advantage of the characteristics of metal magnesium, and as a device using such material. However, it has not been possible to provide metal magnesium particles as a practical material having a stable surface that can be handled even in the atmosphere.

### CITATION LIST

### PATENT LITERATURE

Patent Literature 1: JP 2013-145729
Patent Literature 2: JP 2004-136355
Patent Literature 3: JP 2018-204045
Patent Literature 4: JP 6273633
Patent Literature 5: JP 6273632
Patent Literature 6: JP 5500597

### SUMMARY OF THE INVENTION

### TECHNICAL PROBLEM

In light of these circumstances, the problem of the present invention is to provide nano-size metal magnesium particles which are easy to handle in the atmosphere, and have excellent oxidation resistance, and further in which properties expected of nano-size metal magnesium particles are improved at a maximum, or the properties are controlled by supplementing the properties.

### SOLUTION TO THE PROBLEM

The inventor of the present application has found that nano-size metal magnesium particles which are easy to handle in the atmosphere, and have excellent oxidation resistance, and further in which properties expected of nano-size metal magnesium particles are improved at a maximum, or the properties are controlled by supplementing the properties can be obtained, by coating the nano-size metal magnesium particles with a silicon compound to strictly control the surface state, that is, so as to be silicon compound coated metal magnesium particles in which at least a part of the surface of the nano-size metal magnesium particles is coated with a silicon compound, wherein an average particle diameter of the silicon compound coated metal magnesium particles is 5 nm or more and 500 nm or less, and a thickness of the silicon compound coating is 1 nm or more and 50 nm or less. Thus, the present inventors have completed the present invention.

That is, the present invention is silicon compound coated metal magnesium particles in which at least a part of the surface of the metal magnesium particles is coated with the silicon compound, wherein an average particle diameter of the silicon compound coated metal magnesium particles is 5 nm or more and 500 nm or less, and a thickness of the silicon compound coating is 1 nm or more and 50 nm or less.

Further, the present invention is the silicon compound coated metal magnesium particles described above, wherein the metal magnesium particles in the silicon compound coated metal magnesium particles are not aggregated.

Further, the present invention is any one of the silicon compound coated metal magnesium particles described above, wherein the silicon compound coated metal magnesium particles contain less than 1% by weight of lithium.

Further, the present invention is any one of the silicon compound coated metal magnesium particles described above, wherein the ratio of Si-O bond/Mg-O bond, which is the ratio of Si-O bond to Mg-O bond contained in the silicon compound coated metal magnesium particles, is 0.1 to 2.0,
the Si-O bond is assigned to the peak with the largest area among the peaks in the wavenumber range of 1000 cm⁻¹ to 1300 cm⁻¹ which are obtained by waveform separation of peaks in the wavenumber range of 400 cm⁻¹ to 1400 cm⁻¹, in the infrared absorption spectrum of the silicon compound coated metal magnesium particles measured using the attenuated total reflection method (ATR method) or the diffuse reflection method,
the Mg-O bond is assigned to the peak with the largest area among the peaks in the wavenumber range of 400 cm⁻¹ to 600 cm⁻¹ which are obtained by waveform separation of peaks in the wavenumber range of 400 cm⁻¹ to 1400 cm⁻¹, in the infrared absorption spectrum of the silicon compound coated metal magnesium particles measured using the attenuated total reflection method (ATR method) or the diffuse reflection method,
the ratio of Si-O bond/Mg-O bond is the ratio of the area of the peak assigned to the Si-O bond to the area of the peak assigned to the Mg-O bond, and
the silicon compound coated metal magnesium particles are the silicon compound coated metal magnesium particles in which at least a part of the surface of one metal magnesium particle is coated with the silicon compound.

Further, the present invention is any one of the silicon compound coated metal magnesium particles described above, wherein the silicon compound coated metal magnesium particles contain a hydrocarbon group.

Specifically, the present invention is any one of the silicon compound coated metal magnesium particles described above, which are characterized by the presence of a peak in the wavenumber range of 2500 cm⁻¹ to 3200 cm⁻¹ derived from C-H bond in the infrared absorption spectrum of the silicon compound coated metal magnesium particles measured using the attenuated total reflection method (ATR method) or the diffuse reflection method.

Further, the present invention is any one of the silicon compound coated metal magnesium particles described above, wherein the silicon compound coated metal magnesium particles contain Mg-O-Si bond.

Specifically, the present invention is any one of the silicon compound coated metal magnesium particles described above, which are characterized by the presence of a peak in the wavenumber range of 900 cm⁻¹ to 1000 cm⁻¹ derived from Mg-O-Si bond obtained by waveform separation of the peaks in the wave number range of 400 cm⁻¹ to 1400 cm⁻¹ in the infrared absorption spectrum of the silicon compound coated metal magnesium particles.

Further, the present invention is any one of the silicon compound coated metal magnesium particles described above, wherein an average circularity of the silicon compound coated metal magnesium particles is 0.9 or more.

Further, the present invention is any one of the silicon compound coated metal magnesium particles described above, wherein an ultraviolet-visible (UV-vis) absorption spectrum of a dispersion in which the silicon compound coated metal magnesium particles are dispersed in an organic solvent, shows an absorption peak in the wavelength range of 200 nm to 800 nm.

In addition, the silicon compound coated metal magnesium particles of the present invention can be implemented as a metal magnesium particle surface plasmon absorption medium.

Moreover, the silicon compound coated metal magnesium particles of the present invention can be implemented as a secondary battery electrode composition, a secondary battery electrode using the above composition, or a secondary battery comprising the above secondary battery electrode.

In addition, the silicon compound coated metal magnesium particles of the present invention can be implemented as an implant material or an implant using the above material.

### ADVANTAGEOUS EFFECTS OF THE INVENTION

The present invention provides nano-size metal magnesium particles which are easy to handle in the atmosphere, and have excellent oxidation resistance, and further in which properties expected of nano-size metal magnesium particles are improved at a maximum, or the properties are controlled by supplementing the properties. By using the silicon compound coated metal magnesium particles of the present invention, it is possible to easily design a more accurate composition than conventional compositions for the diversified uses of the nano-size metal magnesium particles and the desired properties thereof.

When the silicon compound coated metal magnesium particles of the present invention are used for a negative electrode of secondary batteries, the surface of the metal magnesium particles has excellent oxidation resistance due to the silicon compound coating, so as to suppress thick growth of oxides or hydroxides on the surface which hinder permeation of electrons and magnesium ions. When an implant material produced by molding the silicon compound coated metal magnesium particles of the present invention is used in the living body, the molded implant material has innumerable boundaries, which become the starting point of the decomposition reaction in the living body, and the decomposition reaction is accelerated more than when the entire implant material is made of metal magnesium or magnesium alloy, and the implant material dissolves quickly and is taken into the living body, so that the burden on the patient decreases. By the silicon compound coated metal magnesium particles of the present invention, an air-stable metal magnesium particle surface plasmon absorbing medium can be provided which can absorb ultraviolet light to visible light by metal magnesium particle surface plasmon.

### BRIEF DESCRIPTION OF THE DRAWINGS

FIG. 1 shows the STEM mapping result of the silicon compound coated metal magnesium particles obtained in Example 1-1.
FIG. 2 shows the result of waveform separation of the peaks in the wavenumber range of 400 cm⁻¹ to 1400 cm⁻¹ in the IR measurement result of the silicon compound coated metal magnesium particles obtained in Example 1-1.
FIG. 3 shows the result of comparing the IR measurement results of the silicon compound coated metal magnesium particles obtained in Example 1-2 and Comparative Example 1 in the wavenumber range of 700 cm⁻¹ to 1200 cm⁻¹.
FIG. 4 shows the IR measurement result of the silicon compound coated metal magnesium particles obtained in Example 1-1 in the wavenumber range of 2500 cm⁻¹ to 3200 cm⁻¹.
FIG. 5 shows the XRD measurement result of the silicon compound coated metal magnesium particles obtained in Example 1-1.
FIG. 6 shows the TEM image of the silicon compound coated metal magnesium particles obtained in Example 4-1.
FIG. 7 shows the STEM image of the silicon compound coated metal magnesium particles obtained in Example 4-2.
FIG. 8 shows the UV-vis absorption measurement result of the silicon compound coated metal magnesium particles obtained in Example 1-3.

### DESCRIPTION OF THE INVENTION

Examples of the embodiments of the present invention will be described below with reference to the drawings. In addition, the embodiments of the present invention are not limited only to the embodiments described below. Further, application examples will be mainly described for a negative electrode material of secondary batteries and an implant, but the applications of the silicon compound coated metal magnesium particles of the present invention are not limited to these embodiments.

### (Silicon compound coated metal magnesium particles - 1)

The silicon compound coated metal magnesium particles of the present invention are silicon compound coated metal magnesium particles in which at least a part of the surface of the metal magnesium particles is coated with the silicon compound, wherein an average particle diameter of the silicon compound coated metal magnesium particles is 5 nm or more and 500 nm or less, and a thickness of the silicon compound coating is 1 nm or more and 50 nm or less. By controlling the surface state of the nano-size silicon compound coated metal magnesium particles, more specifically, by controlling the ratio of Si-O bond/Mg-O bond contained in the silicon compound coated metal magnesium particles, the nano-size metal magnesium particles of the present invention are obtained, which are easy to handle in the atmosphere, and have excellent oxidation resistance, and further in which properties expected of nano-size metal magnesium particles are improved at a maximum, or the properties are controlled by supplementing the properties. The silicon compound coated metal magnesium particles of the present invention are particularly effective for a negative electrode of magnesium ion secondary batteries and an implant material that is implanted in the living body. Since all of these are dissolved as magnesium ions from the particles into the electrolytic solution or the living body, the size of the silicon compound coated metal magnesium particles requires an average particle diameter of 5 nm or more, which allows the particles to maintain their physical size.

In a magnesium ion secondary battery, it is necessary to reversibly perform discharge in which magnesium ions are dissolved as a magnesium negative electrode and charge in which magnesium ions are reduced to metal magnesium particles. It is necessary that magnesium ions are reduced on the surface of the metal magnesium particles. Most of the silicon compound coated metal magnesium particles having the size less than 5 nm are dissolved during discharge, and the state that metal magnesium particles are almost absent during reduction is highly probable, which leads an inconvenience of difficulty of reducing magnesium ions to metal magnesium particles. In addition, when the average particle diameter of the silicon compound coated metal magnesium particles exceeds 500 nm, the dissolution rate of magnesium ions is slow, which hinders rapid discharge in a battery application, resulting in decrease of the discharge current. Therefore, the average particle diameter of the silicon compound coated metal magnesium particles is preferably 5 nm or more and 500 nm or less.

For an in vivo implant material, when the average particle diameter of the silicon compound coated metal magnesium particles for producing an implant material is less than 5 nm, these particles are mixed with the silicon compound coated metal magnesium particles having a larger average particle diameter, and molded into an implant material, and thereby a variation in the grain boundary distribution occurs, which leads an inconvenience that a uniform dissolution rate of a screw, a bolt, etc. for the living body may be changed in the living body. In addition, the silicon compound coated metal magnesium particles with an average particle diameter exceeding 500 nm may remain in the living body even after healing. Therefore, the average particle diameter of the silicon compound coated metal magnesium particles is preferably 5 nm or more and 500 nm or less.

### (Silicon compound coated metal magnesium particles - 2)

In the present invention, the ratio of Si-O bond/Mg-O bond of the silicon compound coated metal magnesium particles is preferably in the range of 0.1 or more and 2.0 or less. More preferably, the ratio is in the range of 0.2 or more and 2.0 or less, and even more preferably, the ratio is in the range of 0.25 or more and 2.0 or less. Thereby, deterioration due to rapid oxidation of metal magnesium particles in the atmosphere can be suppressed. When the ratio of Si-O bond/Mg-O bond of the silicon compound coated metal magnesium particles is less than 0.1, the oxidation resistance thereof is equivalent to that of conventional metal magnesium particles, and makes it difficult to handle in the atmosphere. Further, when the ratio of Si-O bond/Mg-O bond exceeds 2.0, the oxidation resistance of the metal magnesium particles due to the silicon compound coating can be obtained, but when used as a negative electrode of magnesium ion secondary batteries, there is an inconvenience that transfer of electrons and transfer of magnesium ions are greatly impeded during discharge in which magnesium ions are dissolved and charge in which magnesium ions are reduced to metal magnesium particles. Also, when used as an implant material, dissolution of metal magnesium in the living body as magnesium ions is suppressed, so the implant material may remain for a long period of time, and the burden on the living body may increase. Therefore, the preferable ratio of Si-O bond/Mg-O bond is as described above.

### (Form of silicon compound coated metal magnesium particles - 1)

The silicon compound coated metal magnesium particles of the present invention are silicon compound coated metal magnesium particles in which at least a part of the surface of the metal magnesium particles is coated with the silicon compound. The silicon compound coated metal magnesium particles of the present invention are preferably one metal magnesium particle in which at least a part of the surface of the one metal magnesium particle is coated with the silicon compound. The metal magnesium particles may be a magnesium alloy containing different metals as long as they can be dissolved as magnesium ions when applied as a negative electrode of magnesium ion secondary batteries or as an implant material.

### (Form of silicon compound coated metal magnesium particle - 2)

The silicon compound coated metal magnesium particles of the present invention are preferably silicon compound coated metal magnesium particles in which the ratio of Si-O bond/Mg-O bond is controlled, which is the ratio of Si-O bond to Mg-O bond contained in the silicon compound coated metal magnesium particles. Therefore, the silicon compound coated metal magnesium particles of the present invention contain at least silicon (Si) and oxygen (O). A method of evaluating inclusion of silicon (Si) and oxygen (O) is preferably a method of observing a plurality of silicon compound coated metal magnesium particles by a transmission electron microscope (TEM) or a scanning transmission electron microscope (STEM); and confirming the abundance ratio and location of silicon relative to elements other than silicon in each particle by an energy dispersive X-ray spectrometer (EDS). An example thereof includes a method of evaluating the uniformity by determining the abundance ratio (molar ratio) of silicon relative to elements other than silicon contained in one silicon compound coated metal magnesium particle, and calculating the average value and variation coefficient of the molar ratios of a plurality of silicon compound coated metal magnesium particles; and a method of specifying the position of silicon contained in the silicon compound coated metal magnesium particles by mapping. Preferred are silicon compound coated metal magnesium particles in which silicon and oxygen are detected in the vicinity of the surface layer of the silicon compound coated metal magnesium particles in STEM mapping or line analysis. By coating the surface of the metal magnesium particles with the silicon compound, there is an advantage that chemical stability such as oxidation resistance, acid resistance and alkali resistance can be imparted to the metal magnesium particles.

### (Explanation of Si-O bond and Mg-O bond - 1)

In the present invention, various properties of the silicon compound coated metal magnesium particles can be controlled by controlling the ratio of Si-O bond/Mg-O bond, which is the ratio of Si-O bond to Mg-O bond contained in the silicon compound coated metal magnesium particles. The ratio of Si-O bond/Mg-O bond may be determined, for example, by a FT-IR measurement result. Here, IR is an abbreviation for infrared absorption spectroscopy. Hereinafter, it may simply be referred to as an IR measurement. In addition, the ratio of Si-O bond/Mg-O bond may be measured by a method other than IR measurement. An example of the methods includes a method of X-ray photoelectron spectroscopy (XPS), solid nuclear magnetic resonance (solid NMR), electron energy loss spectroscopy (EELS), etc.

### (Explanation of Si-O bond and Mg-O bond - 2)

In the present invention, the ratio of Si-O bond/Mg-O bond of the silicon compound coated metal magnesium particles is preferably obtained by waveform separation of peaks in the wavenumber range of 400 cm⁻¹ to 1400 cm⁻¹ in the IR measurement of the silicon compound coated metal magnesium particles. The peak with the largest area among the peaks derived from Si-O bonds obtained by waveform separation in the wavenumber range of 1000 cm⁻¹ to 1300 cm⁻¹, is preferably assigned to the peak derived from the Si-O bond. Further, the peak with the largest area among the peaks derived from Mg-O bonds obtained by waveform separation in the wavelength range of 400 cm⁻¹ to 600 cm⁻¹, is preferably assigned to the peak derived from Mg-O bond. It is preferable to calculate the ratio of Si-O bond/Mg-O bond as the ratio of the area of the peak assigned to the Si-O bond to the area of the peak assigned to the Mg-O bond.

### (Explanation of thickness of silicon compound coating)

In the present invention, the thickness of the silicon compound coating is 1 nm or more and 50 nm or less. The thickness of the silicon compound coating can be defined by the width of the Si concentration distribution from STEM mapping of Si. When the thickness of the silicon compound coating exceeds 50 nm, the discharge rate which is the dissolution of magnesium ions from metal magnesium particles in a magnesium ion secondary battery, and the charging rate which is due to the reduction of magnesium ions to metal magnesium particles, decrease. Therefore, the thickness of the silicon compound coating is preferably 50 nm or less. In addition, in an implant material, it takes a long time for metal magnesium particles to dissolve into the living body as magnesium ions, and the implant remains in the living body even after healing, so that the burden on the patient increases. In order to prevent the above inconveniences from occurring, the thickness of the silicon compound coating on the metal magnesium particles is preferably 50 nm or less.

When the thickness of the silicon compound coating is less than 1 nm, the oxidation resistance of the metal magnesium particles coated with the silicon compound is not exhibited. The metal magnesium particles are preferably not aggregated, but may be aggregated. Even when a plurality of metal magnesium particles are aggregated, the same effect as in the case of non-aggregated metal magnesium particles can be obtained by setting the thickness of the silicon compound coating to 1 nm or more and 50 nm or less.

### (Mg-O-Si bond)

In Examples, tetrahydrofuran with a residual water content of 10 ppm or less is used as a reaction solvent in order to suppress the reaction of the produced metal magnesium particles with water. Even with a reaction solvent containing 10 ppm of residual water, hydroxyl groups may still exist on the surface of the produced metal magnesium particles. Similarly, tetraethoxysilane which is a silicon compound raw material, can also be hydrolyzed with residual water, and the surface of the silicon compound coating may change to a structure having silanol Si-OH. Therefore, in Examples, the hydroxyl group of the above Mg-OH or Si-OH was reacted with hexamethyldisilazane as shown in the following formula (1), in which the hydrogen atom of the hydroxyl group on the surface of the metal magnesium particle, and the hydrogen atoms of Si-OH on the surface of the silicon compound coated metal magnesium particles are replaced with trimethylsilyl.

M-OH + (Me₃Si)₂NH → M(O-SiMe₃) (1)

wherein M means Mg or Si.

By this substitution reaction, the surface of the silicon compound coated metal magnesium particles is further coated with hydrocarbon groups (methyl, ethyl, etc.) to provide a hydrophobic surface that is stable in the atmosphere, and thereby the adsorption of water in the atmosphere can be suppressed. Therefore, in the present invention, the silicon compound coated metal magnesium particles that are stable in the atmosphere can be also provided further by forming Mg-O-Si bonds on the surface of the silicon compound coated metal magnesium particles to coat the surface with hydrocarbon groups.

When the thickness of the silicon compound coating is less than 1 nm, the formation ratio of Mg-O-Si bond tends to decrease, the area ratio of the opening tends to increase, and the oxidation resistance effect decreases. Therefore, the thickness of the silicon compound coating is preferably 1 nm or more. Also, when the thickness of the silicon compound coating exceeds 50 nm, the transfer speed of magnesium ions passing through the silicon compound coating is affected. Therefore, the thickness of 50 nm or less is appropriate.

In order to improve the oxidation resistance of the metal magnesium particles, the metal magnesium particles are coated with the silicon compound for the purpose of suppressing transfer of oxygen molecules and oxygen ions. When the silicon compound coated metal magnesium particles are used as a negative electrode of magnesium ion secondary batteries, it is preferable that the discharge in which the magnesium ions dissolve from the metal magnesium particles and the charge in which the magnesium ions are reduced to the metal magnesium particles are easily performed. In the present invention, when the silicon compound has a hydrocarbon group, magnesium ions can easily move through openings at the atomic level formed by a composite structure in which an inorganic silica structure and a hydrocarbon group exist in mixture. Since the radius of magnesium ion is 0.072 nm, one of oxygen molecule is 0.35 nm, and one of water molecule (H₂O) is 0.38 nm, by the silicon compound coating of the metal magnesium particles, the permeation of oxygen molecules and water molecules can be suppresses more than magnesium ions, and thereby improving the oxidation resistance. In the present invention, when the silicon compound contains a hydrocarbon group, that is, when it contains a CH bond such as a methyl group or a methylene group included in the hydrocarbon group, the silicon compound has a hydrophobic property against permeation of water molecules, and water molecules cannot easily move in the silicon compound coating. Hydrocarbon groups include, for example, C₁₋₆ alkyl groups and the like, preferably methyl, ethyl and the like.

When the silicon compound coated metal magnesium particles of the present invention are used as a negative electrode of magnesium ion secondary batteries, by coating the surface of the metal magnesium particles susceptible to oxidation with the silicon compound, it is made difficult that they are oxidized, and the discharge in which the magnesium ions easily pass through the silicon compound coating and dissolve, and the charge in which the magnesium ions are reduced to the metal magnesium particles can be performed. Therefore, by controlling the silicon compound coating state, the silicon compound does not completely coat the surface of the metal magnesium particles and hinder the transfer of magnesium ions.

### (Explanation of amorphous silicon compound)

In the present invention, the silicon compound that coats at least a part of the surface of the metal magnesium particles preferably contains an amorphous silicon oxide. Thereby, the ratio of Si-O bond/Mg-O bond can be easily controlled. A method of evaluating whether the silicon compound contains amorphous silicon oxide, is not particularly limited. But, the method includes a method of evaluation in combination of confirming the presence of Si and O by the above STEM mapping, and confirming the presence of silicon oxide by IR measurement, and confirming that no peak derived from crystalline silica (SiO₂) in XRD measurement; and a method of confirming that no crystal lattice is observed at the site where Si and O are detected in TEM observation or STEM observation; and the like.

### (Method of producing the silicon compound coated metal magnesium particles: Preferable method)

As an example of a method of producing the silicon compound coated metal magnesium particles of the present invention, preferred is a method of producing the silicon compound coated metal magnesium particles, which comprises the step of providing a metal magnesium raw material liquid containing at least a raw material of the metal magnesium particles to be coated with the silicon compound, and a metal magnesium reduction liquid containing at least a metal magnesium reducing substance for precipitating the metal magnesium particles; the step of reacting in a mixed fluid obtained by mixing the metal magnesium raw material liquid and the metal magnesium reduction liquid and precipitating the metal magnesium particles; and the step of mixing the above mixed fluid containing the precipitated metal magnesium particles and a silicon compound raw material liquid containing at least a raw material of the silicon compound as the coating compound, and coating at least a part of the surface of the metal magnesium particles with the silicon compound.

### (Metal magnesium raw material)

The metal magnesium raw material is not particularly limited. Any metal magnesium raw material may be used as long as the silicon compound coated metal magnesium particles can be produced by a method of such as reaction and precipitation. Hereinafter, compounds of magnesium may be collectively referred to as the compound. The compound is not particularly limited, but examples thereof include a salt of magnesium and an organic compound containing magnesium, etc. These compounds of magnesium may be used alone or in combination of two or more. In the present invention, when the metals constituting the silicon compound coated metal magnesium particles are a plurality of different metal elements or semi-metal elements, the ratio of M2 to M1 (M2/M1) is preferably 0.1 or less, wherein the main metal magnesium is M1 and the secondary metal or semi-metal element is M2.

### (Metal magnesium reducing substance)

The metal magnesium reducing substance is not particularly limited as long as the substance can precipitate the metal magnesium raw material as metal magnesium particles.

For example, it is preferable to use a reducing agent capable of reducing magnesium ions contained in the metal magnesium raw material liquid. An examples thereof includes metal lithium, metal sodium, metal potassium, metal cesium, metal rubidium, etc., which are elements belonging to the alkali metal in the periodic table. These metals can be used alone or in combination as a mixture.

Metal lithium and a condensed aromatic compound can be used as the metal magnesium reducing substance. In such case, the number of moles of metal lithium required is at least twice that of the metal magnesium raw material. The condensed aromatic compound is a compound whose oxidation-reduction potential after electron transfer from metal lithium is more base (-) than the oxidation-reduction potential of a magnesium ion, and includes naphthalene, biphenyl, and the like. In an organic solvent such as tetrahydrofuran, in the presence of the condensed aromatic compound, metal lithium dissolves and an electron is transferred to the condensed aromatic compound. In Examples, a metal magnesium reduction liquid containing metal lithium and naphthalene at a molar ratio of 1:1 is prepared and reacted with the metal magnesium raw material liquid.

Journal of Materials Chemistry, Vol. A2, p. 9718 (2014) describes that metal magnesium particles were produced by reacting metal lithium, naphthalene of a condensed aromatic compound, and a metal magnesium raw material simultaneously. In this production method, a large amount of metal lithium is used, which is 4.6 to 9.2 times higher than the molar amount of metal magnesium raw material. In such case, a very high concentration of magnesium lithium alloy is obtained, in which the metal lithium concentration in the produced metal magnesium particles is 16.8% by weight to 30.1% by weight.

When the molar ratio of metal lithium and naphthalene of the condensed aromatic compound is 1:1, a naphthalene anion which is a one-electron reductant of naphthalene, is generated, and the electron is transferred from the naphthalene anion to the magnesium raw material. It is confirmed that metal magnesium particles can be produced even when the molar ratio of metal lithium and naphthalene is 1:0.5. However, at the above molar ratio of 1 :0.5, naphthalene dianion which is two electron reductant of naphthalene, is produced and 1-ethyl-1,2-dihydronaphthalene or 1-ethyl-1,4-dihydronaphthalene is generated. To remove this inconvenience, the molar ratio of metal lithium and naphthalene is preferably 1: 1.

### (Silicon compound raw material)

Examples of the silicon compound raw material include an oxide and a hydroxide of silicon, a compound such as a salt and an alkoxide of silicon, and a hydrate thereof. Although not particularly limited, examples of the silicon compound raw material include a silicate such as sodium silicate, phenyltrimethoxysilane, methyltrimethoxysilane, methyltriethoxysilane, 3-glycidoxypropyltrimethoxysilane, 3-trifluoropropyl-trimethoxysilane, methacryloxypropyltriethoxysilane, tetramethoxysilane (TMOS), tetraethoxysilane (TEOS), and an oligomeric condensate of TEOS, such as ethylsilicate 40, tetraisopropylsilane, tetrapropoxysilane, tetraisobutoxysilane, tetrabutoxysilane , triethylchlorosilane (TECS), and a similar material thereof. Furthermore, another siloxane compound, bis(triethoxysilyl)methane, 1,9-bis(triethoxysilyl)nonane, diethoxydichlorosilane, triethoxychlorosilane, etc. may be used as the silicon compound raw material. Furthermore, a silylating agent may be used together with the silicon compound raw material. The silylating agent may be one that reacts with a hydroxyl group to form a trihydrocarbon group silyl ether, etc. Examples of the silylating agent include a trihydrocarbon group silyl halide (e.g. triethylchlorosilane (TECS), trimethylchlorosilane, triethylbromosilane, etc.), and a hexahydrocarbon group disilazane (e.g. hexamethyldisilazane (HMDS), hexaethyldisilazane, etc.). Preferable silylating agent includes hexamethyldisilazane (HMDS).

### (Solvent)

As a solvent used when precipitating the silicon compound coated metal magnesium particles of the present invention, any solvent can be used as long as it can produce the silicon compound coated metal magnesium particles of the present invention. Examples of the solvent include preferably an inert solvent, more preferably an ether solvent and the like. Examples of an ether solvent include diethyl ether, tetrahydrofuran, 1,3-dioxolane, 1,4-dioxane, dimethoxyethane, diethylene glycol dimethyl ether, etc. Among these solvents, tetrahydrofuran is preferred because it has an ignition point of 321°C and can be handled safely. Also, the residual water content is preferably 10 ppm or less so that metal magnesium produced by reduction is not dissolved by the residual water and a large amount of magnesium hydroxide is not produced.

### (Dispersing agent, etc.)

Various dispersing agents or surfactants may be used according to the purpose and need, as long as they do not adversely affect the production of the silicon compound coated metal magnesium particles of the present invention. Although not particularly limited, various commonly used commercial products, products, or newly synthesized products can be used as the dispersing agent and surfactant. Examples thereof include a dispersing agent such as an anionic surfactant, a cationic surfactant, a nonionic surfactant, and various polymers. These may be used alone or in combination of two or more. The surfactant and dispersing agent described above may be contained in at least one fluid of the metal magnesium raw material liquid and the metal magnesium reduction liquid. Further, the surfactant and dispersing agent described above may be contained in another fluid different from the metal magnesium raw material liquid and the metal magnesium reduction liquid.

### (Method of producing silicon compound coated metal magnesium particles: Outline of the method)

In the step of coating at least a part of the surface of the metal magnesium particles with the silicon compound, the metal magnesium particles are preferably coated with the silicon compound before the metal magnesium particles aggregate. When the silicon compound raw material liquid is mixed with the fluid containing the metal magnesium particles, it is important that the silicon compound raw material liquid is added at a rate faster than the precipitated metal magnesium particles aggregate, and the silicon compound precipitates on the surface of the metal magnesium particles. If the fluid containing the metal magnesium particles is prepared and then the silicon compound raw material liquid is added to the fluid, the concentrations of the fluid containing the metal magnesium particles and the silicon compound raw material gradually change, and a state in which the metal magnesium particles tend to disperse becomes a state in which they tend to aggregate, and the silicon compound precipitates on the surface of the aggregated metal magnesium particles, resulting in possibility of failing to exhibit the properties of the present invention. Therefore, it is preferable to allow the silicon compound raw material contained in the silicon compound raw material liquid to act immediately after the metal magnesium particles are precipitated. It is preferable to obtain the silicon compound coated metal magnesium particles by a method that between two processing surfaces being capable of approaching to and separating from each other and rotating relative to each other as described in Patent Literature 6, the metal magnesium particles are precipitated, and then the surface of the metal magnesium particles are continuously coated by the silicon compound. There is an advantage that production of the silicon compound coated metal magnesium particles of the present invention, control of the ratio of Si-O bond/Mg-O bond, and control of the properties of the silicon compound coated metal magnesium particles can be strictly controlled by changing the temperature or conditions when obtaining the silicon compound coated metal magnesium particles.

### (Method of producing silicon compound coated metal magnesium particles: Apparatus)

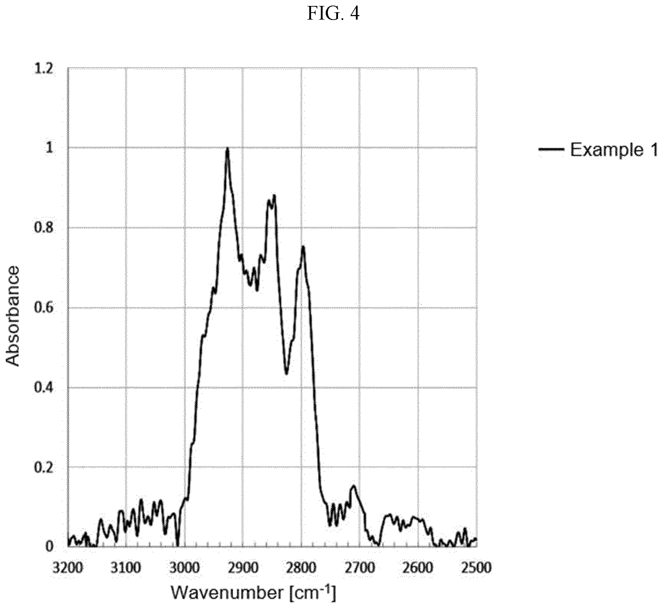

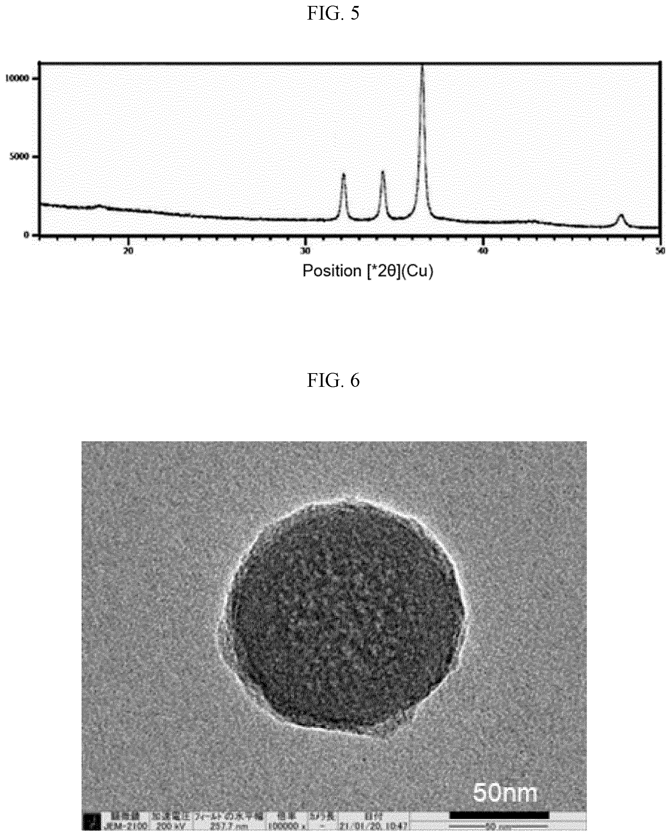

An example of a method of producing the silicon compound coated metal magnesium particles of the present invention includes, for example, a method of producing the silicon compound coated metal magnesium particles by using a microreactor, or by a reaction in a batch vessel under a dilute system and the like. In order to produce the silicon compound coated metal magnesium particles, the apparatus and method as proposed by the present applicant and described in JP 2009-112892 may be also used. The apparatus described in JP 2009-112892 comprises a stirring tank having an inner peripheral surface which cross-section is circular, and a mixing tool attached to the stirring tank with a slight gap to the inner peripheral surface of the stirring tank, wherein the stirring tank comprises at least two fluid inlets and at least one fluid outlet; from one of the fluid inlets, the first fluid to be processed containing one of the reactants among the fluids to be processed is introduced into the stirring tank; from one fluid inlet other than the above inlet, the second fluid to be processed containing one of reactants different from the above reactant is introduced into the stirring tank through a different flow path; at least one of the stirring tank and the mixing tool rotates at a high speed relative to the other to let the above fluids be in a state of a thin film; and in the above thin film, the reactants contained in the first and second fluids to be processed are reacted. JP 2009-112892 further describes that three or more inlet tubes may be provided as shown in FIG. 4 and 5 to introduce three or more fluids to be processed into the stirring tank. An example of the microreactor is an apparatus based on the same principle as the fluid processing apparatus described in Patent Literature 6.

In the method of producing silicon compound coated metal magnesium particles of the present invention, a method different from the above production method includes a method of treating particles of a substance that can be a precursor of metal magnesium contained in the metal magnesium particles in a reducing atmosphere. Specifically, an example thereof is a method of preparing the silicon compound coated metal magnesium precursor particles in which at least a part of the surface of the particles containing the metal magnesium precursor is coated with a silicon compound, and of treating the silicon compound coated metal magnesium precursor particles in a reducing atmosphere. Examples of the precursor of metal magnesium include various compounds such as oxides and hydroxides containing magnesium element constituting the metal magnesium particles, and hydrates and organic solvates thereof. For example, the silicon compound coated metal magnesium precursor particles can be reduced to the silicon compound coated metal magnesium particles by heat-treating them in a hydrogen atmosphere, which is a reducing atmosphere. In addition, even if the silicon compound is also reduced to silicon by treating the silicon compound coated metal magnesium precursor particles in a reducing atmosphere, it is possible that the treatment in an atmosphere such as the air can give a silicon compound such as silicon oxide, to obtain the silicon compound coated metal magnesium particles in the same manner as described above. Thus, there is an advantage that production of the silicon compound coated metal magnesium particles of the present invention, control of the ratio of Si-O bond/Mg-O bond, and control of the ratio of Si-O bond/Mg-O bond can be performed substantially at the same time, and in addition the ratio of Si-O bond/Mg-O bond can be more strictly controlled, by treating the silicon compound coated metal magnesium precursor particles containing the precursor of the metal magnesium particles in a reducing atmosphere.

The silicon compound coated metal magnesium particles of the present invention can also be obtained by treating the silicon doped metal magnesium precursor particles in which silicon is contained in the precursor, in a reducing atmosphere. For example, at first, silicon doped magnesium hydroxide particles which are silicon doped metal magnesium precursor particles, are heat-treated in a reducing atmosphere to reduce both silicon and magnesium to obtain silicon magnesium alloy particles. Next, the obtained silicon magnesium alloy particles are treated in an oxidizing atmosphere such as the air to oxidize the silicon contained in the surface of the silicon magnesium alloy particles to form a silicon compound such as silicon oxide. Thereby, the silicon compound coated metal magnesium particles such as silicon compound coated metal magnesium particles or silicon compound coated silicon magnesium alloy particles can be produced.

When the silicon compound coated metal magnesium precursor particles or the silicon doped metal magnesium precursor particles are reduced to obtain the silicon compound coated metal magnesium particles, the particle diameter of the silicon compound coated metal magnesium precursor particles is preferably 500 nm or less. When the particle diameter is 500 nm or less, uniform reduction treatment to give the silicon compound coated metal magnesium particles is possible, and there is an advantage that the ratio of Si-O bond/Mg-O bond can be controlled at the same time. In addition, there is an advantage that metal magnesium particles can be produced by reduction according to the present invention even for metal magnesium which has been difficult to be reduced by methods such as electroreduction using large energy. The method of producing the silicon compound coated metal magnesium precursor particles are not particularly limited, but in the same manner as the silicon compound coated metal magnesium particles, the silicon compound coated metal magnesium precursor particles may be produced by a production method by using the apparatus described in Patent Literature 6, or a method of pulverization by using a bead mill or the like, followed by coating the metal magnesium precursor particles with a silicon compound in a reaction vessel or using the microreactor or the like.

### EXAMPLE

Hereinafter, the present invention is explained in more detail with reference to Examples, but the present invention is not limited only to these Examples.

### (Preparation of samples for TEM observation and preparation of samples for STEM observation)

The silicon compound coated metal magnesium particles obtained in Examples and Comparative Examples were dispersed in a dispersion medium, and the obtained dispersion was dropped to a Formvar support film, and dried to prepare a sample for TEM observation or a sample for STEM observation.

### (Transmission electron microscope and energy dispersive X-ray spectrometer: TEM-EDS analysis)

For observation and quantitative analysis of the silicon compound coated metal magnesium particles by TEM-EDS analysis, the transmission electron microscopy JEM-2100 (JEOL Ltd.) equipped with the energy dispersive X-ray analyzer JED-2300 (JEOL Ltd.) was used. The observation condition was the acceleration voltage of 200 kV, and the observation magnification of 10,000 times or more. The particle diameters were calculated from the maximum distance between two points on the outer periphery of the silicon compound coated metal magnesium particles observed by TEM, and the average value (average particle diameter) of the measured particle diameters of 100 particles was calculated. The molar ratios of the elemental components constituting the silicon compound coated metal magnesium particles were calculated by TEM-EDS, and the average value of the molar ratios calculated for 50 or more particles was calculated.

### (Scanning transmission electron microscope and energy dispersive X-ray spectrometer: STEM-EDS analysis)

For the mapping and quantification of elements contained in the silicon compound coated metal magnesium particles by STEM-EDS analysis, the atomic resolution analytical electron microscopy JEM-ARM200F (JEOL Ltd.) equipped with the energy dispersive X-ray analyzer Centurio (JEOL Ltd.) was used. The observation condition was the acceleration voltage of 200 kV and the observation magnification of 50,000 times or more, and a beam diameter of 0.2 nm was used for analysis.

### (X-ray diffraction measurement)

For the X-ray diffraction (XRD) measurement, the powder X-ray diffractometer Empyrean (Spectris Co., Ltd., PANalytical Division) was used. The measurement condition was measurement range of 10 to 100 [°2Theta], Cu anticathode, tube voltage of 45 kV, tube current of 40 mA, and scanning speed of 0.3 °/min. The XRD was measured using the dry powder of the silicon compound coated metal magnesium particles obtained in Examples.

### (IR measurement)

For the IR measurement, the Fourier transform infrared spectrophotometer FT/IR-6600 (JASCO Corporation) was used. The measurement condition was the resolution of 4.0 cm⁻¹ and accumulated number of 128 times, using an ATR method or diffuse reflection method. Waveform separation of peaks in the wavenumber range of 400 cm⁻¹ to 1400 cm⁻¹ in the IR spectrum is performed by using the spectrum analysis program attached to the control software of the above FT/IR-6600, and curve fitting so that the residual sum of squares between IR spectrum synthesized by waveform separated peaks (synthesis Data) and the IR spectrum of the measured Example was 0.05 or less. Measurements were performed using dry powders of the silicon compound coated metal magnesium particles produced in Examples and Comparative Examples.

### (ICP-OES measurement)

The concentrations of magnesium and lithium in the silicon compound coated metal magnesium particles were measured using ICP-OES (high frequency inductively coupled plasma emission spectrometer, ICPS-8100, Shimadzu Corporation). The measurement conditions were as follows: 4 mg of the silicon compound coated metal magnesium particles were weighed for a sample, and dissolved in nitric acid, and dissolved by heating on a hot plate at 60°C for 1 hour; all the mixture was transferred to a 25 mL volumetric flask and a constant volume was obtained by adding pure water; 5 mL of this solution was taken and diluted to 50 mL with 10% by volume nitric acid to quantify lithium, and then the sample solution was further diluted with pure water to quantify magnesium. A calibration curve was prepared using a magnesium standard solution for atomic absorption spectrometry (Kanto Chemical Co., Ltd.) and a lithium standard solution for atomic absorption spectrometry (Kanto Chemical Co., Ltd.). An emission line of 670.784 nm was used for lithium, and an emission line of 279.553 nm was used for magnesium.

### (UV-vis absorption spectrum measurement)

The UV-vis (ultraviolet-visible) absorption spectrum of the silicon compound coated metal magnesium particles was measured using an ultraviolet-visible near-infrared spectrophotometer (V-770, JASCO Corporation). The measurement range was 200 nm to 900 nm, the sampling rate was 0.2 nm, and the measurement speed was low. A liquid cell with a thickness of 1 mm was used for the measurement. For the UV-vis absorption spectrum of the silicon compound coated metal magnesium particles, a measurement solution was used which was obtained by weighing 0.5 mg of the silicon oxide coated metal magnesium particles in a wet cake state, and adding it to 5 mL of tetrahydrofuran (THF), followed by ultrasonic dispersion for 30 minutes.

### (Measurement of Circularity)

The circularity of the silicon compound coated metal magnesium particles of Example 4 was measured as follows: the image obtained by TEM was approximated to ellipse by using the software iTEM for TEM image (Olympus Soft Imaging Solutions, GmbH); next, the major axis (D), perimeter (L) and area (S) of the ellipse which is the projected image of the silicon compound coated metal magnesium particles were determined from the analysis results of the TEM image analysis software; and 4πS/L² was calculated using these values to obtain the circularity. As the circularity value is closer to 1, the particle is closer to a spherical shape. When the shape of the particle is truly spherical, the circularity is maximum 1. The average circularity was the arithmetic mean of the circularities calculated for 50 independent silicon compound coated metal magnesium particles.

### (Measurement of thickness of silicon compound coating)

The thickness of the silicon compound coating of the silicon compound coated metal magnesium particles was measured by specifying the width of the coated portion from the light-dark contrast of the TEM observation image or the STEM-HAADF image of the particles observed by TEM or STEM. The light-dark contrast of the image observed by TEM or STEM is generated depending on the degree to which electrons are partially scattered by interaction with the sample as electrons pass through the sample. Since the scattered electrons increase in the high electron density region of the sample, the transmission image of the electron beam observed in the high electron density region is observed to be dark. Since a metal has a high electron density, the transmission image of the electrons passing through the metal magnesium particles in the silicon compound coated metal magnesium particles is observed to be darker than that of the silicon compound due to scattering. The silicon compound coating is an insulator due to the silicon compound containing the hydrocarbon, so that the electrons passing through the silicon compound coated region are scattered less than that passing through the metal magnesium particles. Accordingly, the thickness of the silicon compound coating can be distinguished from the metal magnesium particles, by being observed to be brighter than the metal magnesium particles. The thickness of the silicon compound coating was measured as follows: with respect to the width of the coated region, four locations including the locations visually determined to be the maximum width and minimum width (for particles where only a part of the surface of the metal magnesium particle is coated with the silicon compound, the minimum width was set to 0) were selected; the arithmetic average of the measured four values was taken as the thickness of the silicon compound coating of a single silicon compound coated metal magnesium particle; similarly, the widths of the coated parts were determined for 50 particles; and the arithmetic mean value thereof was taken as the thickness of the silicon compound coating in each Example. The width of the silicon compound coated part may be obtained from the contrast distribution of a silicon (Si) mapping image by STEM or line analysis.

### (Example 1)

Using the fluid processing apparatus based on the principle described in Patent Literature 6, the ratio of Si-O bond/Mg-O bond contained in the silicon compound coated metal magnesium particles was controlled to be 0.1 to 2.0 to produce the silicon compound coated metal magnesium particles. The resulting silicon compound coated metal magnesium particles exhibited oxidation resistance.

As a reaction solvent, super dehydrated tetrahydrofuran with a residual water content of 10 ppm or less (FUJIFILM Wako Pure Chemical Corporation) was used. A metal magnesium raw material liquid (Liquid A), a metal magnesium reduction liquid (Liquid B), and a silicon compound raw material liquid (Liquid C) were prepared in a glove box in an argon atmosphere. Specifically, Table 1 shows Examples 1-1 to 1-4. Based on the formulation of the metal magnesium raw material liquid, respective components were homogeneously mixed by stirring for 60 minutes with a magnetic stirrer at a preparation temperature of 25 °C to prepare the metal magnesium raw material liquid. In addition, based on the formulation of the metal magnesium reduction liquid, respective components of the metal magnesium reduction liquid were stirred with a magnetic stirrer at a preparation temperature of 25 °C for 120 minutes to completely dissolve metal lithium to prepare the metal magnesium reduction liquid. Furthermore, based on the formulation of the silicon compound raw material liquid, respective components of the silicon compound raw material liquid were uniformly mixed by stirring with a magnetic stirrer at a preparation temperature of 25 °C for 60 minutes to prepare the silicon compound raw material liquid.

Regarding the substances indicated by chemical formulas or abbreviations in Table 1, MgBr₂ is magnesium bromide (Strem Chemicals, Inc.), Li is metal lithium (Kishida Chemical Co., Ltd.), C₁₀H₈ is naphthalene (Kanto Chemical Co., Ltd.), TEOS is tetraethylorthosilicate (FUJIFILM Wako Pure Chemical Corporation), and HMDS is hexamethyldisilazane (Tokyo Chemical Industry Co., Ltd.).

**[Table 1]**

| Example | Liquid A (Metal magnesium raw material liquid) | | Liquid B (Metal magnesium reduction liquid) | | | | Liquid C (Silicon compound raw material liquid) | | | |
|---|---|---|---|---|---|---|---|---|---|---|
| | Raw material | (mol/L) | Raw material | (mol/L) | Raw material | (mol/L) | Raw material | (mol/L) | material | (mol/L) |
| 1-1 | MgBr₂ | 0.1 | Li | 0.2 | C₁₀H₈ | 0.2 | TEOS | 0.1 | HMDS | 0.1 |
| 1-2 | MgBr₂ | 0.1 | Li | 0.2 | C₁₀H₈ | 0.2 | TEOS | 0.2 | HMDS | 0.1 |
| 1-3 | MgBr₂ | 0.1 | Li | 0.2 | C₁₀H₈ | 0.2 | TEOS | 0.3 | HMDS | 0.1 |
| 1-4 | MgBr₂ | 0.1 | Li | 0.2 | C₁₀H₈ | 0.2 | TEOS | 0.4 | HMDS | 0.1 |

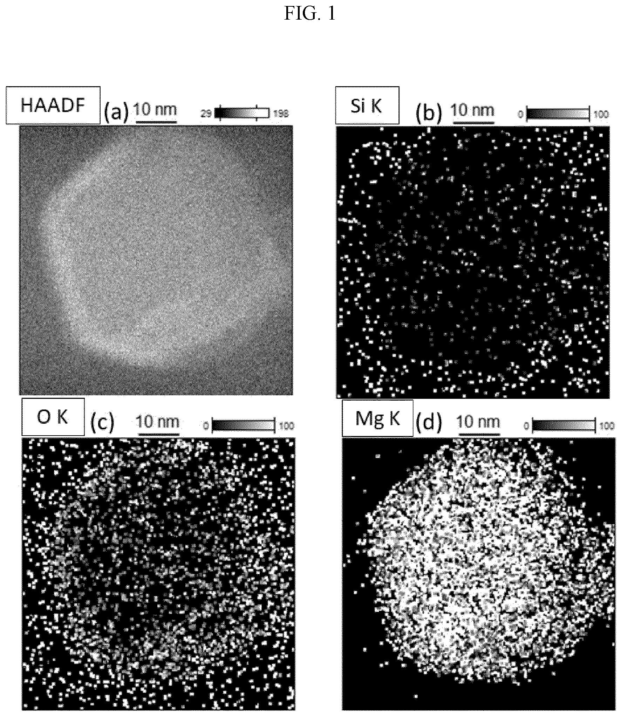

Next, the prepared metal magnesium raw material liquid (Liquid A), the metal magnesium reduction liquid (Liquid B), and the silicon compound raw material liquid (Liquid C) were mixed using the fluid processing apparatus described in Patent Literature 6 by the applicant of the present application. Here, the fluid processing apparatus described in Patent Literature 6 is the apparatus described in FIG. 1(B) which has a concentric circular ring shape surrounding the central opening of the processing surface 2 which is a ring shape disk wherein the openings d20 and d30 of the second and third introduction parts are formed in a ring shape. Specifically, Liquid A was introduced between the processing surfaces 1 and 2 from the first introduction part d1, and while the processing part 10 was operated at a rotation speed of 1700 rpm, Liquid B was introduced between the surfaces 1 and 2 from the second introduction part d2 to mix the metal magnesium raw material liquid and the metal magnesium reduction liquid in the thin film fluid, and the metal magnesium particles were precipitated between the processing surfaces 1 and 2. Next, Liquid C was introduced between the processing surfaces 1 and 2 from the third introduction part d3 and mixed with the mixed fluid containing metal magnesium particles in the thin film fluid. The silicon compound was precipitated on the surface of the metal magnesium particles, and the discharge liquid containing the silicon compound coated metal magnesium particles (hereinafter referred to as the silicon compound coated metal magnesium particle dispersion) was discharged from between the processing surfaces 1 and 2 of the fluid processing apparatus. The discharged silicon compound coated metal magnesium particle dispersion was recovered in a beaker through the vessel.

Table 2 shows the operation conditions of the fluid processing apparatus. Here, the introduction temperature (liquid sending temperature) and introduction pressure (liquid sending pressure) of Liquid A, Liquid B and Liquid C shown in Table 2 were measured using a thermometer and a pressure gauge provided in the sealed introduction paths (the first introduction part d1, the second introduction part d2, and the third introduction part d3) between the processing surfaces 1 and 2. The introduction temperature of Liquid A shown in Table 2 was the actual temperature of Liquid A in the first introduction part d1 under the introduction pressure. The introduction temperature of Liquid B was the actual temperature of Liquid B in the second introduction part d2 under the introduction pressure. The introduction temperature of Liquid C was the actual temperature of Liquid C in the third introduction part d3 under the introduction pressure.

**[Table 2]**

| Example | Disk rotation number (rpm) | Introduction flow rate (liquid sending flow rate) | | | Introduction temperature (liquid sending temperature) | | | Introduction pressure (liquid sending pressure) | | |
|---|---|---|---|---|---|---|---|---|---|---|
| | | (mL/min) | | | (°C) | | | (MPaG) | | |
| | | Liquid A | Liquid B | Liquid C | Liquid A | Liquid B | Liquid C | Liquid A | Liquid B | Liquid C |
| 1-1 | 1700 | 20 | 20 | 40 | 25 | 25 | 25 | 0.1 | 1-2 | <0.1 |
| 1-2 | 1700 | 30 | 30 | 40 | 25 | 25 | 25 | 0.1 | 1-2 | <0.1 |
| 1-3 | 1700 | 40 | 40 | 40 | 25 | 25 | 25 | 0.1 | 1-2 | <0.1 |
| 1-4 | 1700 | 50 | 50 | 40 | 25 | 25 | 25 | 0.1 | 1-2 | <0.1 |

A dry powder sample and a wet cake sample were prepared from the silicon compound coated metal magnesium particle dispersion discharged from the fluid processing apparatus and recovered in a beaker. The preparation method was performed according to a conventional method of this kind of treatment. The discharged silicon compound coated metal magnesium particle dispersion was recovered, and the silicon compound coated metal magnesium particles were sedimented by centrifugation (7000 rpm, 30 minutes), and the supernatant was removed. After the removal, ultrasonic washing with tetrahydrofuran and sedimentation were repeated, and a part of the wet cake of the finally obtained silicon compound coated metal magnesium particles was dried at -0.10 MPaG at 25 °C for 20 hours to obtain a dry powder sample. The remainder was used as a wet cake sample.

### (Comparative Example 1)

The silicon compound coated metal magnesium particles were produced in the same manner as in Example 1-1, except that the silicon compound raw material liquid of Liquid C did not contain HMDS.

FIG. 1 shows the results of elemental mapping of the silicon compound coated metal magnesium particles obtained in Example 1-1 using STEM. In FIG. 1, (a) shows a dark field image (HAADF image), (b) shows the mapping result of silicon (Si), (c) shows the mapping result of oxygen (O), and (d) shows the mapping result of magnesium (Mg). As seen in FIG. 1, the silicon compound coated metal magnesium particles obtained in Example 1-1 were observed as the metal magnesium particles entirely covered with the silicon compound. The same STEM mapping results as in Example 1-1 were obtained for the silicon compound coated metal magnesium particles obtained in Examples 1-2 to 1-4. Regarding Example 1-4, the silicon compound coated metal magnesium particles in which a part of the surface of the metal magnesium particles was coated with the silicon compound containing silicon oxide were also confirmed, which were not the metal magnesium particles entirely covered with the silicon compound. According to the present invention, it is possible to provide the silicon compound coated metal magnesium particles in which at least a part of the surface of the metal magnesium particles is coated with the silicon compound.

By the elemental analysis by TEM-EDS of the silicon compound coated metal magnesium particles produced in Example 1-1, it was confirmed that the silicon compound coated metal magnesium particles had an atomic % ratio of Mg and Si of 97.7:2.3. Table 3 shows the results of similar calculations of Examples 1-2 to 1-4 and Comparative Example 1. The silicon compound coated metal magnesium particles produced in Examples 1-2 to 1-4 exhibited similar particle shapes and similar results in the elemental analysis of Mg and Si. The atomic % ratio of Mg and Si in the silicon compound coated metal magnesium particles produced in Comparative Example 1 was 99.5:0.5.

Also, when the thickness of the silicon compound coating was calculated by the method described above, the average thickness of the silicon compound coating in Example 1-1 was 14 nm. Table 3 shows the results of similar calculations of Examples 1-2 to 1-4 and Comparative Example 1. It was confirmed that the thickness of the silicon compound coating of the silicon compound coated metal magnesium particles of Examples 1-1 to 1-4 was 1 nm or more and 50 nm or less of the silicon compound coated metal magnesium particles. The average thickness of the silicon compound coating of the silicon compound coated metal magnesium particles of Comparative Example 1 was 0.8 nm and less than 1 nm.

**[Table 3]**

| | Average particle diameter (nm) | Ratio of Si-O bond/Mg-O bond | Atomic % | | Silicon compound coating thickness (nm) |
|---|---|---|---|---|---|
| | | | Mg | Si | |
| Example 1-1 | 55 | 0.26 | 97.7 | 2.3 | 4 |
| Example 1-2 | 57 | 0.38 | 96.6 | 3.4 | 6 |
| Example 1-3 | 60 | 0.43 | 96.2 | 3.8 | 7 |
| Example 1-4 | 60 | 0.51 | 95.8 | 4.2 | 8 |
| Comparative Example 1 | 55 | 0.06 | 99.5 | 0.5 | 0.8 |

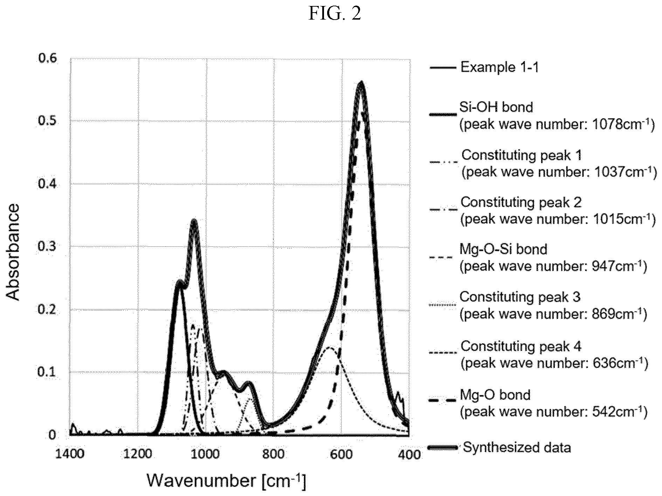

FIG. 2 shows the result of waveform separation among the peaks in the wavenumber range of 400 cm⁻¹ to 1400 cm⁻¹ in the IR measurement result of the silicon compound coated metal magnesium particles obtained in Example 1-1. In addition, an IR spectrum synthesized from respective waveform separated peaks is shown as synthesized data. Since the residual sum of squares is 0.05 or less, the measured IR spectrum of Example 1-1 and the IR spectrum of the synthesized data match well, and it is confirmed that the IR spectra are sufficiently overlapped. As can be seen in FIG. 2, as a result of waveform separation in the wavenumber range of 400 cm⁻¹ to 1400 cm⁻¹ in Example 1-1, the peak with the largest area among the peaks derived from Si-O bond separated in the wavenumber range of 1000 cm⁻¹ to 1300 cm⁻¹ was assigned to the peak derived from Si-O bond. The peak with the largest area among the peaks present in the wavenumber range of 400 cm⁻¹ to 600 cm⁻¹ was assigned to the peak derived from Mg-O bond. The ratio of Si-O bond/Mg-O bond was calculated from the area of the peak derived from Si-O bond and the area of the peak derived from Mg-O bond, and the ratio in Example 1-1 was 0.26. The ratios of Si-O bond/Mg-O bond in Examples 1-2 to 1-4 and Comparative Example 1 were also calculated in the same manner. The results are shown in Table 3. The ratios of Si-O bond/Mg-O bond in Examples 1-2 to 1-4 were 0.38 to 0.51. The ratio of Si-O bond/Mg-O bond in Comparative Example 1 was 0.06.

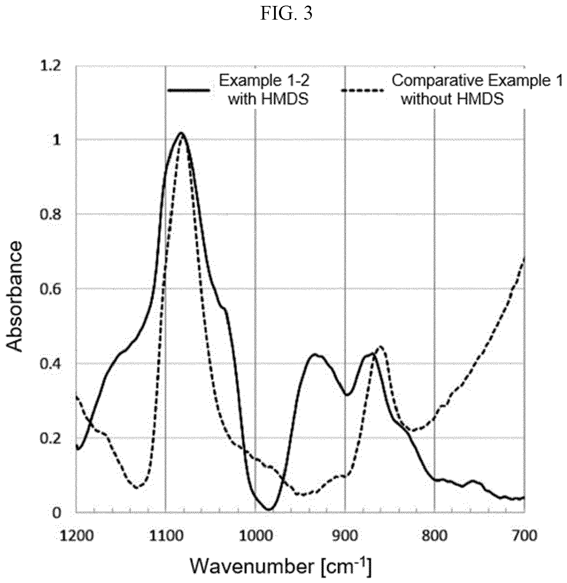

FIG. 3 shows the IR measurement results of the silicon compound coated metal magnesium particles of Example 1-2 produced by mixing HMDS and the silicon compound coated metal magnesium particles of Comparative Example 1 not mixed with HMDS. It is known that M-O-Si bond in which a metal atom (M) and a silicon atom are bonded through an oxygen atom exhibits an absorption peak in the wavenumber range of 900 cm⁻¹ to 1000 cm⁻¹ in the IR spectrum. In Example 1-2 mixed with HMDS, an absorption peak at 940 cm⁻¹ was observed, and in Comparative Example 1, no absorption peak was observed at 940 cm⁻¹. Therefore, the silicon compound coated metal magnesium particles of Example 1-2, produced by mixing HMDS, exhibited the absorption peak of Mg-O-Si bond at 940 cm⁻¹ within the wavenumber range of 900 cm⁻¹ to 1000 cm⁻¹ in the IR spectrum, and thereby it was confirmed that Mg-O-Si bond derived from HMDS was included.

FIG. 4 shows the IR measurement result of the silicon compound coated metal magnesium particles obtained in Example 1-1 in the wavenumber range of 2500 cm⁻¹ to 3200 cm⁻¹. As seen in FIG. 4, in the present IR measurement results, the peak appearing in the range of 2700 cm⁻¹ to 2800 cm⁻¹ is attributed to O=C-H bond, and the peaks appearing in the range of 2800 cm⁻¹ to 2900 cm⁻¹ are attributed to symmetric stretching vibration of -CH₂-(methylene) and symmetric stretching vibration of -CH₃ (methyl), and the peaks appearing in the range of 2900 cm⁻¹ to 3000 cm⁻¹ are attributed to antisymmetric stretching vibration of - CH₂- (methylene) and degenerate stretching vibration of -CH₃ (methyl). From this result, the IR measurement result of the metal magnesium silicon compound particles of the present invention exhibited the absorption peaks derived from C-H bond in the wavenumber range of 2500 cm⁻¹ to 3200 cm⁻¹, and thereby it was confirmed that the silicon compound coated metal magnesium particles of the present invention contained a hydrocarbon group.

FIG. 5 shows the XRD measurement result of the silicon compound coated metal magnesium particles obtained in Example 1-1. As seen in FIG. 5, only the peak derived from Mg was detected in the XRD measurement. That is, it was confirmed that the silicon compound containing silicon oxide having Si-O bond confirmed by the TEM and IR measurements was a silicon compound existing in an amorphous state. Similar XRD measurement results were obtained for Examples 1-2 to 1-4.

The silicon compound coated metal magnesium particles obtained in Examples 1-1 to 1-4 showed no diffraction peak from magnesium hydroxide in the XRD measurement, and thereby it was confirmed that they were stable in the atmosphere. In the silicon compound coated metal magnesium particles produced in Comparative Example 1, hydroxide due to oxidation of the metal magnesium particles was confirmed by XRD, so that the oxidation resistance of the metal magnesium particles was not satisfactory. For this reason, when the thickness of the silicon compound coating of the silicon compound coated metal magnesium particles is less than 1 nm, they cannot be stably handled in the atmosphere, and thereby it was confirmed that there was a problem with their use as a negative electrode of magnesium ion secondary batteries or an implant.

### (Example 2)

In Example 2, the silicon compound coated metal magnesium particles were produced by changing the concentration of HMDS as the silicon compound raw material in the silicon compound coated metal magnesium particles. As a reaction solvent, super dehydrated tetrahydrofuran with a residual water content of 10 ppm or less (FUJIFILM Wako Pure Chemical Corporation) was used. The production conditions are shown in Table 4. The same conditions as in Example 1-1 described in Table 2 were used for the operation conditions of the fluid processing apparatus.

**[Table 4]**

| Example | Liquid A (Metal magnesium raw material liquid) | | Liquid B (Metal magnesium reduction liquid) | | | | Liquid C (Silicon compound raw material liquid) | | | |
|---|---|---|---|---|---|---|---|---|---|---|
| | Raw material | (mol/L) | Raw material | (mol/L) | Raw material | (mol/L) | Raw material | (mol/L) | Raw material | (mol/L) |
| 2-1 | MgBr₂ | 0.1 | Li | 0.2 | C₁₀H₈ | 0.2 | TEOS | 0.2 | HMDS | 0.2 |
| 2-2 | MgBr₂ | 0.1 | Li | 0.2 | C₁₀H₈ | 0.2 | TEOS | 0.3 | HMDS | 0.3 |
| 2-3 | MgBr₂ | 0.1 | Li | 0.2 | C₁₀H₈ | 0.2 | TEOS | 0.4 | HMDS | 0.4 |
| 2-4 | MgBr₂ | 0.1 | Li | 0.2 | C₁₀H₈ | 0.2 | TEOS | 0.6 | HMDS | 0.6 |

Table 5 shows the analysis results of the obtained silicon compound coated metal magnesium particles.

**[Table 5]**

| Example | Average particle diameter (nm) | Ratio of Si-O bond/Mg-O bond | Atomic % | | Silicon compound coating thickness (nm) |
|---|---|---|---|---|---|
| | | | Mg | Si | |
| 2-1 | 58 | 0.23 | 98.0 | 2.0 | 3 |
| 2-2 | 60 | 0.33 | 97.2 | 2. 8 | 5 |
| 2-3 | 63 | 0.42 | 96.6 | 3.4 | 6 |
| 2-4 | 70 | 0.55 | 95.5 | 4.5 | 8 |

### (Example 3)

The silicon compound coated metal magnesium particles were produced using triethylchlorosilane (TECS) which was a halogenated silane, instead of TEOS as a silicon compound raw material. Triethylchlorosilane (Tokyo Chemical Industry Co., Ltd.) was used as TECS. As a reaction solvent, super dehydrated tetrahydrofuran with a residual water content of 10 ppm or less (FUJIFILM Wako Pure Chemical Corporation) was used. The production conditions are shown in Table 6. The operation conditions of the fluid processing apparatus in Examples 3-1 and 3-2 were the same as in Examples 1-1 and 1-2 described in Table 2, respectively.

**[Table 6]**

| Example | Liquid A (Metal magnesium raw material liquid) | | Liquid B (Metal magnesium reduction liquid) | | | | Liquid C (Silicon compound raw material liquid) | | | |
|---|---|---|---|---|---|---|---|---|---|---|
| | Raw material | (mol/L) | Raw material | (mol/L) | Raw material | (mol/L) | Raw material | (mol/L) | Raw material | (mol/L) |
| 3-1 | MgBr₂ | 0.1 | Li | 0.2 | C₁₀H₈ | 0.2 | TECS | 0.2 | HMDS | 0.2 |
| 3-2 | MgBr₂ | 0.1 | Li | 0.2 | C₁₀H₈ | 0.2 | TECS | 0.4 | HMDS | 0.4 |

Table 7 shows the analysis results of the obtained silicon compound coated metal magnesium particles.

**[Table 7]**

| Example | Average particle diameter (nm) | Ratio of Si-O bond/Mg-O bond | Atomic % | | Silicon compound coating thickness (nm) |
|---|---|---|---|---|---|
| | | | Mg | Si | |
| 3-1 | 58 | 0.30 | 97.3 | 2.7 | 5 |
| 3-2 | 63 | 0.49 | 96.0 | 4.0 | 7 |

The results of TEM and XRD of the silicon compound coated metal magnesium particles obtained in Examples 2 and 3 were similar to those of Example 1. In both cases, only the peak derived from metal magnesium was detected in the XRD measurement results, and thereby it was confirmed that they were stable in the atmosphere. In addition, it was confirmed that the silicon compound containing silicon oxide confirmed by the TEM and IR measurements was a silicon compound existing in an amorphous state.

The silicon compound coated metal magnesium particles produced by changing the concentration of HMDS as the silicon compound raw material shown in Table 4, and the silicon compound coated metal magnesium particles produced by using TECS (triethylchlorosilane) instead of TEOS in the silicon compound raw material liquid shown in Table 6, have average particle diameters in the range of 50 nm to 70 nm and the ratios of Si-O bond/Mg-O bond in the range of 0.1 to 0.6 as shown in Tables 5 and 7. The thickness of the silicon compound coating of the silicon compound coated metal magnesium particles was 1 nm or more and 50 nm or less.

### (Example 4)

A metal magnesium raw material liquid (Liquid A), a metal magnesium reduction liquid (Liquid B), and a silicon compound raw material liquid (Liquid C) were prepared under the conditions shown in Table 8. Liquid A, Liquid B and Liquid C were mixed using the fluid processing apparatus described in Patent Literature 6 by the applicant of the present application. Here, the fluid processing apparatus described in Patent Literature 6 is the apparatus described in FIG. 1(B) which has a concentric circular ring shape surrounding the central opening of the processing surface 2 which is a ring shape disk wherein the openings d20 and d30 of the second and third introduction parts are formed in a ring shape. Specifically, Liquid A was introduced between the processing surfaces 1 and 2 from the first introduction part d1, and while the processing part 10 was operated at a rotation speed of 1700 rpm, Liquid B was introduced between the surfaces 1 and 2 from the second introduction part d2 to mix the metal magnesium raw material liquid and the metal magnesium reduction liquid in the thin film fluid, and the metal magnesium particles were precipitated between the processing surfaces 1 and 2. Next, Liquid C was introduced between the processing surfaces 1 and 2 from the third introduction part d3 and mixed with the mixed fluid containing metal magnesium particles in the thin film fluid. The silicon compound was precipitated on the surface of the metal magnesium particles, and the discharge liquid containing the silicon compound coated metal magnesium particles was discharged from between the processing surfaces 1 and 2 of the fluid processing apparatus. The discharged silicon compound coated metal magnesium particle dispersion was recovered in a beaker through the vessel.

**[Table 8]**

| Example | Liquid A (Metal magnesium raw material liquid) | | Liquid B (Metal magnesium reduction liquid) | | | | Liquid C (Silicon compound raw material liquid) | | | |
|---|---|---|---|---|---|---|---|---|---|---|
| | Raw material | (mol/L) | Raw material | (mol/L) | Raw material | (mol/L) | Raw material | (mol/L) | Raw material | (mol/L) |
| 4-1 | MgBr₂ | 0.1 | Li | 0.2 | C₁₀H₈ | 0.2 | TEOS | 1.0 | HMDS | 1.0 |
| 4-2 | MgBr₂ | 0.1 | Li | 0.2 | C₁₀H₈ | 0.2 | TEOS | 2.0 | HMDS | 2.0 |

The silicon compound coated metal magnesium particle dispersion prepared as described above was transferred to a separately prepared flask (200 mL), connected to a Dimroth reflux condenser, and heated in an oil bath. In this Example, tetrahydrofuran (THF) was used as a solvent for all Liquid A, Liquid B and Liquid C, and when a temperature reached 66 °C of the boiling point of THF (under an atmospheric pressure), from the Dimroth reflux condenser the liquid THF droplets generated by cooling the THF vapor were dripped into the mixed fluid in which the silicon compound coated metal magnesium particles were dispersed in the flask. After such state started, the mixture was heated for 1 hour. After heating under reflux for 1 hour, the dispersion of the silicon compound coated metal magnesium particles was collected in a beaker.

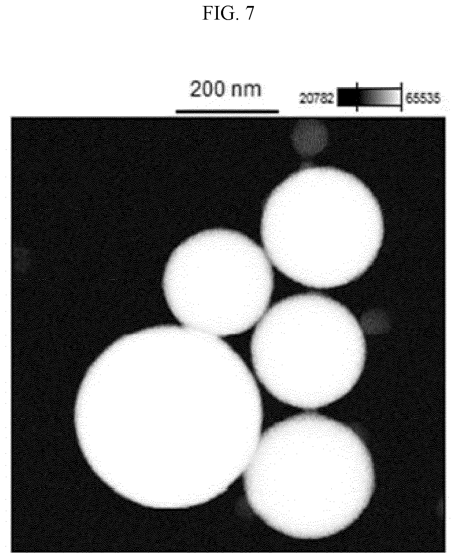

FIG. 6 shows a TEM observation image of the silicon compound coated metal magnesium particles produced in Example 4-1. The atomic % ratio of Mg and Si evaluated by TEM-EDS was 90.2:9.8. The IR measurement confirmed Mg-O-Si bond at 947 cm⁻¹. FIG. 7 shows the spherical silicon compound coated metal magnesium particles produced in Example 4-2 observed by STEM-EDS.

Table 9 shows the analysis results of the obtained silicon compound coated metal magnesium particles.

**[Table 9]**

| Example | Average particle diameter (nm) | Ratio of Si-O bond/Mg-O bond | Atomic % | | Silicon compound coating thickness (nm) | Average circularity |
|---|---|---|---|---|---|---|
| | | | Mg | Si | | |
| 4-1 | 180 | 1.25 | 90.2 | 9.8 | 18 | 0.94 |
| 4-2 | 250 | 1.14 | 90.5 | 9.5 | 16 | 0.93 |

It was confirmed that the thickness of the silicon compound coating of the silicon compound coated metal magnesium particles was 1 nm or more and 50 nm or less.

The circularity of the silicon compound coated metal magnesium particles produced in Example 4-1 was analyzed using the method described above for 50 particles existing independently. As a result, 50 silicon compound coated metal magnesium particles had an average particle diameter of 180 nm and an average circularity of 0.94. The average circularity of Example 4-1 and that of Example 4-2 are shown in Table 9. When circularity of the silicon compound coated metal magnesium particles is high, in case of using them for a negative electrode of magnesium ion secondary batteries, the silicon compound coated metal magnesium particles having a substantially spherical shape contact each other with a point contact, resulting in the existence of inevitable gaps between these substantially spherical particles. Due to the existence of gaps, the penetration of the electrolyte is easy, and the entire surface of a single particle can be used for the transfer of magnesium ions and electrons, resulting in the highest current density, i.e., the highest available energy density. In addition, when the spherical silicon compound coated metal magnesium particles are used as an implant material and compressed into pellets, the grain boundaries derived from the original silicon compound coated metal magnesium particles are uniformly distributed, and thereby there is an advantage that the dissolution rate of metal magnesium in the living body becomes constant, and non-uniformity of the dissolution rate of metal magnesium is reduced.

When the lithium concentration of the silicon compound coated metal magnesium particles produced in the present invention was measured by ICP-OES, the lithium concentrations in Examples 1 to 4 were confirmed to be 0.1 % by weight to 0.6 % by weight, and were confirmed to be less than 1% by weight. From these results, it was confirmed that the metal magnesium particles of the silicon compound coated metal magnesium particles produced in the present invention were highly pure metal magnesium particles, although a slight amount of lithium was found to be mixed therein. Regarding lithium remaining in the metal magnesium particles, when the silicon compound coated metal magnesium particles are used for a negative electrode of magnesium ion secondary batteries, while the metal magnesium particles dissolve as magnesium ions during discharge, lithium ions are also generated, and while these magnesium ions are reduced to the metal magnesium particles during charge, lithium ions are also reduced, and thereby there is concern about the occurrence of morphological abnormalities in which dendrites which are dendritic crystals, are generated. In the reduction of magnesium ions to metal magnesium particles, safe use is possible without the risk of dendrite formation in the present invention. However, in the negative electrode of the metal magnesium particles in which lithium ions are reduced to metal lithium, there is possibility that the dendrite of metal lithium breaks through the separator that suppresses contact with the positive electrode in the magnesium ion secondary battery, causing a short circuit with the positive electrode, and problems in terms of long term stable use. Therefore, it is preferable to ensure that the excellent reducing properties of magnesium ions are not inhibited by the coexisting lithium. In an implant material, it has been reported that there is concern about the occurrence of diseases due to an increase in the concentration of lithium in the living body. Therefore, it is preferable to reduce the amount of lithium present in metal magnesium particles as much as possible.

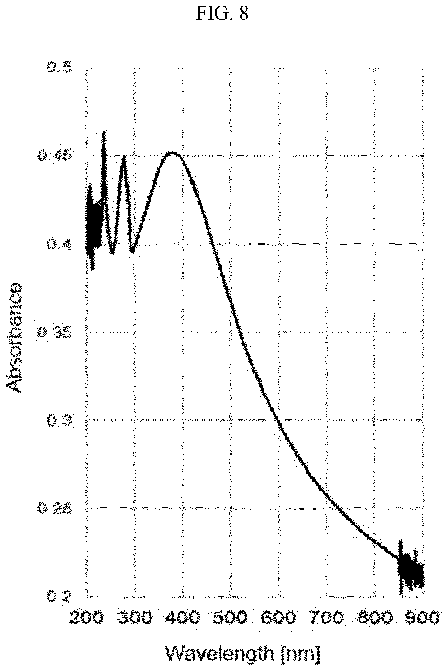

FIG. 8 shows the UV-vis absorption spectrum of the silicon compound coated metal magnesium particles in Example 1-3, which was measured after ultrasonically dispersed in tetrahydrofuran for 30 minutes. The absorption maximum wavelengths are confirmed as two maximum peaks at 200 nm to 300 nm and a peak at 380 nm, and the absorption spectrum spreads over the visible light wavelength range of 500 nm to 600 nm. Therefore, it was confirmed that absorption by surface plasmons of the obtained silicon oxide coated metal magnesium particles is possible from ultraviolet light to visible light. Similarly, absorption from ultraviolet light to visible light was obtained in other Examples, and it was confirmed that the silicon compound coated metal magnesium particles of the present invention have absorption from ultraviolet light to visible light. Since a light over a wide range from ultraviolet light to visible light can be used, the silicon compound coated metal magnesium particles of the present invention may be applied as a photocatalyst responsive to visible light for indoor use. In addition, by taking the advantage of this characteristic, it is now possible to apply to Raman scattering and infrared absorption spectrometry, where optical sensing materials mainly composed of a precious metal such as gold and silver have conventionally been used.

## Claims

1. Silicon compound coated metal magnesium particles in which at least a part of a surface of the metal magnesium particles is coated with the silicon compound,
wherein an average particle diameter of the silicon compound coated metal magnesium particles is 5 nm or more and 500 nm or less, and
a thickness of the silicon compound coating is 1 nm or more and 50 nm or less.

2. The silicon compound coated metal magnesium particles according to claim 1, wherein the metal magnesium particles in the silicon compound coated metal magnesium particles are not aggregated.

3. The silicon compound coated metal magnesium particles according to claim 1 or 2, wherein the silicon compound coated metal magnesium particles contain less than 1% by weight of lithium.

4. The silicon compound coated metal magnesium particles according to any one of claims 1 to 3, wherein the ratio of Si-O bond/Mg-O bond, which is the ratio of Si-O bond to Mg-O bond contained in the silicon compound coated metal magnesium particles, is 0.1 to 2.0,
the Si-O bond is assigned to the peak with the largest area among the peaks in the wavenumber range of 1000 cm⁻¹ to 1300 cm⁻¹ which are obtained by waveform separation of peaks in the wavenumber range of 400 cm⁻¹ to 1400 cm⁻¹, in the infrared absorption spectrum of the silicon compound coated metal magnesium particles measured using the attenuated total reflection method (ATR method) or the diffuse reflection method,
the Mg-O bond is assigned to the peak with the largest area among the peaks in the wavenumber range of 400 cm⁻¹ to 600 cm⁻¹ which are obtained by waveform separation of peaks in the wavenumber range of 400 cm⁻¹ to 1400 cm⁻¹, in the infrared absorption spectrum of the silicon compound coated metal magnesium particles measured using the attenuated total reflection method (ATR method) or the diffuse reflection method,
the ratio of Si-O bond/Mg-O bond is the ratio of the area of the peak assigned to the Si-O bond to the area of the peak assigned to the Mg-O bond, and
the silicon compound coated metal magnesium particles are the silicon compound coated metal magnesium particles in which at least a part of the surface of one metal magnesium particle is coated with the silicon compound.

5. The silicon compound coated metal magnesium particles according to any one of claims 1 to 4, wherein the silicon compound coated metal magnesium particles contain a hydrocarbon group.

6. The silicon compound coated metal magnesium particles according to any one of claims 1 to 5, wherein the silicon compound coated metal magnesium particles contain Mg-O-Si bond.

7. The silicon compound coated metal magnesium particles according to any one of claims 1 to 6, wherein an average circularity of the silicon compound coated metal magnesium particles is 0.9 or more.

8. The silicon compound coated metal magnesium particles according to any one of claims 1 to 7, wherein an ultraviolet-visible (UV-vis) absorption spectrum of a dispersion in which the silicon compound coated metal magnesium particles are dispersed in an organic solvent, shows an absorption peak in the wavelength range of 200 nm to 800 nm.

9. A secondary battery electrode composition comprising the silicon compound coated metal magnesium particles according to any one of claims 1 to 8, or a secondary battery electrode using the composition.

10. A secondary battery comprising the secondary battery electrode according to claim 9 .

11. An implant material comprising the silicon compound coated metal magnesium particles according to any one of claims 1 to 8, or an implant using the material.
